# DEMANDE DE BREVET EUROPEEN

(11) **EP 4 111 950 A1**
(43) Date de publication de la demande: **04.01.2023**
(21) Numéro de dépôt: 22180765.4
(22) Date de dépôt: 23.06.2022
(51) Int. Cl.: A61B 5/00, A61B 5/384

(54) **SYSTÈME ET PROCÉDÉ DE SURVEILLANCE**

(30) Priorité: 01.07.2021 FR 2107149
(71) Demandeur: Fondation A de Rothschild, 75019 Paris (FR)
(72) Inventeur: HUBERFELD, Gilles, 92380 GARCHES (FR); BOURDILLON, Pierre Vincent Adrien, 75004 PARIS (FR)
(74) Mandataire: Cabinet Beau de Loménie

(57) **Abrégé**

Système de surveillance d'un patient ainsi qu'un procédé de surveillance d'un patient utilisant un tel système, le système de surveillance comprenant au moins un capteur (11), configuré pour être posé sur le patient (3) et pour enregistrer au moins un signal relatif au patient (3), et
un robot (4), comportant
- un dispositif de locomotion (24), commandé par un module de pilotage (37),
- au moins une caméra (26),
- un premier module de communication (31), configuré pour recevoir, de manière sans fil, le signal dudit au moins un capteur (11),
- un deuxième module de communication (32), configuré pour communiquer avec un serveur distant (61), et
- un module de traitement (36), configuré pour traiter le signal dudit au moins un capteur (11) et fournir un signal traité au deuxième module de communication (32).

## Description

### Domaine Technique

Le présent exposé concerne un système de surveillance d'un patient ainsi qu'un procédé de surveillance d'un patient utilisant un tel système. Un tel système et un tel procédé sont particulièrement utiles pour réaliser la surveillance d'un patient sur plusieurs jours, y compris en dehors d'un environnement médicalisé. Une telle surveillance peut notamment être utile pour l'examen et/ou l'accompagnement de patients épileptiques.

### Technique antérieure

L'électroencéphalographie (EEG) est une technique d'enregistrement de l'activité des neurones du système nerveux central, leur activation produisant des champs électriques captés à la surface du cuir chevelu à l'aide d'électrodes.

Dans le champ de l'épilepsie, les activités neuronales anormalement générées par le cerveau épileptique sont enregistrées par l'EEG. Dans la plupart des cas, l'enregistrement des activités neuronales dites interictales, c'est-à-dire entre les crises, suffit à établir le diagnostic d'épilepsie et à déterminer le type d'épilepsie dont souffre le patient.

Toutefois, chez certains patients, l'enregistrement des activités interictales n'est pas suffisant et les crises d'épilepsies elles-mêmes doivent être enregistrées. Cela est notamment le cas lorsqu'aucune anomalie interictale n'est présente (cela concerne jusqu'à 20% des patients). Cela est également le cas lorsque les patients continuent à présenter des crises malgré les traitements médicamenteux (cela concerne de 20 à 30 % des patients) : les crises doivent alors être enregistrées de manière à valider le diagnostic et surtout à identifier la zone cérébrale initiant les crises si une solution de traitement chirurgical est envisagée. L'enregistrement prolongé est également proposé aux patients pour lesquels il existe un doute diagnostic devant des malaises, l'enregistrement EEG et de la vidéo des malaises permettant le diagnostic positif de l'épilepsie et différentiel d'autres types de malaises.

Dans tous ces cas, les activités EEG de crise épileptique doivent enregistrées et corrélées aux manifestations cliniques des patients. Or, dans la mesure où les crises sont peu fréquentes et non prévisibles, il est nécessaire de prolonger les enregistrements EEG sur plusieurs jours afin de parvenir à enregistrer un ou plusieurs crises. Des électrodes d'enregistrement sont collées sur le cuir chevelu du patient et l'activité électrique cérébrale est recueillie en continu tandis que le patient est filmé. L'exploration dure de plusieurs jours à plusieurs semaines, jusqu'à ce que les crises recueillies soient informatives.

A ce jour, ces enregistrements ont lieu en milieu hospitalier. Ils nécessitent donc d'équiper une chambre hospitalière en systèmes EEG et vidéo, avec des connections centralisées et une équipe de surveillance. Un tel système est donc compliqué et onéreux à mettre en place. Le nombre de patients suivis est également contraint par le nombre de chambres équipées, ce qui limite fortement les capacités de diagnostic et de suivi de l'équipe médicale.

Il existe donc un réel besoin pour un système de surveillance de patient et un procédé de surveillance de patient qui soient dépourvus, au moins en partie, des inconvénients inhérents à la méthode connue précitée.

### Exposé de l'invention

Le présent exposé concerne un système de surveillance d'un patient, comprenant
au moins un capteur, configuré pour être posé sur le patient et pour enregistrer
au moins un signal relatif au patient, et
un robot, comportant
   - un dispositif de locomotion, commandé par un module de pilotage,
   - au moins une caméra,
   - un premier module de communication, configuré pour recevoir, de manière sans fil, le signal dudit au moins un capteur,
   - un deuxième module de communication, configuré pour communiquer avec un serveur distant, et
   - un module de traitement, configuré pour traiter le signal dudit au moins un capteur et fournir un signal traité au deuxième module de communication.

Grâce à un tel robot muni d'une caméra et d'un module de traitement et capable de communiquer avec au moins un capteur posé sur le patient, il est possible de surveiller le patient de manière beaucoup plus facile, pratique et agile.

En particulier, le robot peut être fourni et introduit dans n'importe quel environnement : ainsi, il n'est plus nécessite d'équiper et d'immobiliser un espace dédié, en particulier une chambre d'hôpital. De cette manière, il est par exemple possible de surveiller le patient à domicile, ce qui augmente le confort du patient et réduit l'occurrence de biais liés au contexte hospitalier.

L'utilisation d'un tel robot permet également de se soustraire à la contrainte du foncier : le nombre de patient surveillés n'est donc plus limité par le nombre de chambres équipées disponibles. Le coût d'un tel système est également fortement réduit par rapport au coût d'équipement et d'immobilisation d'une chambre en hôpital.

De plus, grâce à son dispositif de locomotion, le robot est capable de se déplacer dans l'environnement du patient afin, par exemple, de le suivre de pièce en pièce. La liberté de mouvement du patient est ainsi augmentée : en particulier, le patient peut continuer ses activités quotidiennes comme à son habitude, ce qui est plus confortable pour le patient et réduit encore le risque de biais dans le comportement et les symptômes du patient.

L'utilisation d'une connexion sans-fil entre le ou les capteurs et le robot permet une autonomie de déplacement du patient par rapport au robot : le patient peut ainsi continuer ses activités normales en faisant abstraction de la présence du robot.

Outre l'envoi du signal traité au serveur distant permettant à une équipe médicale d'analyser le signal du ou des capteurs en vue par exemple de poser un diagnostic, le robot peut également servir d'interface vidéo et/ou audio entre le patient et l'équipe médicale. Le robot peut ainsi constituer, pour certains patients, une présence rassurante, symbole du lien avec l'équipe médicale de surveillance. En particulier, le robot peut se comporter à la manière d'un robot compagnon, permettant par exemple de rassurer le patient durant une crise. Il permet de plus à l'équipe médicale d'interagir avec le patient, notamment à l'occasion d'une crise.

Dans certains modes de réalisation, ledit au moins un capteur est un ensemble de plusieurs capteurs, chaque capteur étant configuré pour être posé sur le patient et pour enregistrer au moins un signal relatif au patient. Plusieurs signaux, de mêmes natures ou de natures différentes, peuvent ainsi être enregistrés, ce qui augmente la quantité d'informations à la disposition de l'équipe médicale pour poser le diagnostic.

Dans certains modes de réalisation, au moins un capteur est une électrode d'électroencéphalogramme, de préférence du type cupule. En effet, ce système de surveillance est tout particulièrement adapté pour réaliser des électroencéphalogrammes (EEG), notamment de longue durée et, en particulier, des vidéo-électroencéphalogrammes, notamment pour des patients épileptiques ou souffrants de mouvements anormaux ou de troubles du sommeil.

Dans certains modes de réalisation, ledit au moins un capteur comprend au moins un jeu d'électrodes d'électroencéphalogramme comportant au moins 8 électrodes et de préférence au moins 32 électrodes.

Dans certains modes de réalisation, au moins un capteur est un cardiofréquencemètre. La fréquence cardiaque du patient peut en effet être une donnée utile dans certaines applications, par exemple les troubles du sommeil ou l'épilepsie, la fréquence cardiaque augmentant le plus souvent pendant les crises.

Dans certains modes de réalisation, au moins un capteur est une électrode d'électrocardiogramme. Le système peut en effet être utile pour suivre l'activité cardiaque d'un patient dans son environnement habituel.

Dans certains modes de réalisation, au moins un capteur est un capteur de mouvement, par exemple un accéléromètre. Un tel capteur de mouvement peut notamment être posé sur un membre supérieur, le ventre, les jambes, le menton ou encore à proximité des yeux du patient. Ce type de capteur peut être utile pour l'étude du sommeil du patient ou lorsque le patient présente des mouvements anormaux, par exemple épileptiques.

Dans certains modes de réalisation, au moins un capteur est un débitmètre. Un tel débitmètre peut notamment être posé sur, dans ou à proximité du nez du patient afin de mesurer le flux d'air de la respiration du patient. Une telle donnée peut notamment être utile pour étudier le sommeil du patient.

Dans certains modes de réalisation, au moins un capteur est un microphone. Un tel capteur peut être utile pour enregistrer les ronflements du patient ou encore pour déterminer le flux d'air de la respiration du patient. De telles données peuvent notamment être utiles pour étudier le sommeil du patient. Toutefois, de préférence, ledit au moins un capteur comprend au moins un capteur qui n'est pas un microphone.

Dans certains modes de réalisation, au moins un capteur est un oxymètre. Un tel oxymètre peut notamment être posé à l'extrémité d'un doigt du patient ou sur le lobe de l'une de ses oreilles. Le taux de saturation en oxygène du patient est notamment utile pour étudier le sommeil du patient, ainsi que pour certaines crises d'épilepsie.

Dans certains modes de réalisation, au moins un capteur est un appareil de mesure des résistances cutanées sympathiques. Un tel dispositif peut notamment être posé à l'extrémité de deux doigts du patient. La mesure des résistances cutanées sympathiques est notamment utile pour étudier le stress du patient, ainsi que suite à certaines crises d'épilepsie, le risque de mort subite.

Dans certains modes de réalisation, au moins un capteur est un appareil de mesure de l'activité électromyographique. Un tel dispositif peut notamment être posé sur n'importe que muscle du patient. La mesure de l'activité électromyographique est notamment utile pour étudier l'activation motrice au cours de crises épileptiques, de mouvements anormaux, ou du sommeil.

Dans certains modes de réalisation, le système comprend un boîtier de communication connecté audit au moins un capteur et configuré pour communiquer avec le premier module de communication du robot. Un tel boîtier est particulièrement utile lorsque plusieurs capteurs sont présents afin de centraliser l'ensemble des signaux des capteurs avant leur transmission au robot. Ce boîtier est de préférence porté par le patient, par exemple dans une sacoche en bandoulière. Il peut être alimenté en électricité par des piles ou des batteries rechargeables.

Dans certains modes de réalisation, le boîtier de communication est configuré pour formater le signal dudit au moins un capteur avant de le transmettre au premier module de communication du robot. Ce formatage peut notamment comprendre la numérisation du signal et/ou sa conversion dans un autre format. Il peut également comprendre le regroupement de plusieurs signaux provenant de plusieurs capteurs. De préférence, le boîtier de communication ne réalise aucun autre traitement que ce formatage ; en particulier, il ne réalise aucune analyse.

Dans certains modes de réalisation, le premier module de communication est configuré pour recevoir le signal dudit au moins un capteur par wifi. Ce type de connexion est en effet particulièrement fiable pour la transmission de données à l'échelle locale.

Dans certains modes de réalisation, le robot comprend un premier système informatique, incluant le premier module de communication et le module de traitement, et un deuxième système informatique, distinct du premier système informatique, incluant le module de pilotage. Une telle configuration est avantageuse pour que le processus de contrôle du robot d'une part et le processus de traitement du signal d'autre part soient indépendants l'un de l'autre et puissent être exécutés en parallèle sans risque que l'un des processus ne retarde l'exécution de l'autre processus. En effet, ces deux processus nécessitent des ressources importantes.

Dans certains modes de réalisation, ces deux systèmes informatiques fonctionnent sous deux systèmes d'exploitation différents : cela permet de s'adapter à différents types d'équipements présentant des compatibilités préférentielles avec des systèmes d'exploitation différents. Par exemple, le premier système informatique peut fonctionner sous Windows tandis que le deuxième système informatique peut fonctionner sous Android. Ces deux systèmes d'exploitation peuvent communiquer.

Dans certains modes de réalisation, le robot comprend un châssis, le deuxième système informatique étant intégré à l'intérieur du châssis tandis que le premier système informatique est rapporté sous le châssis ou sur le côté du châssis, par exemple dans son dos. En particulier, le deuxième système informatique peut être le système informatique natif du robot tandis que le premier système informatique lui est rajouté pour faire fonctionner le module de traitement. De cette manière, il est possible d'utiliser un robot préexistant comme plateforme de base à laquelle sont ajoutées les fonctions supplémentaires permettant la surveillance du patient, en particulier le module de traitement : la conception du robot est ainsi facilitée.

Dans certains modes de réalisation, le robot possède un centre de gravité situé à une hauteur du sol inférieure à 60 cm. De cette manière, on réduit le risque que le robot ne bascule sur le côté et ne soit donc endommagé ou ne puisse plus assurer sa locomotion, en cas d'erreur de déplacement ou en cas de choc avec le patient, par exemple lors d'une crise de ce dernier.

Dans certains modes de réalisation, le dispositif de locomotion comprend des roues dont au moins une est motrice.

Dans certains modes de réalisation, le dispositif de locomotion comprend au moins deux pieds articulés. Le robot peut notamment être bipède ou quadrupède. En particulier, grâce à de tels pieds articulés, le robot est capable de franchir des marches et, si besoin, de gravir un escalier afin, par exemple, de suivre le patient dans différents étages d'un bâtiment : ceci est tout particulièrement utile lorsque le domicile du patient est une maison comprenant plusieurs étages.

Dans certains modes de réalisation, le robot comprend un dispositif de spatialisation lui permettant de détecter son environnement. Un tel dispositif de spatialisation permet au robot de se repérer dans son environnement et, ainsi, d'éviter les obstacles. Ce dispositif de spatialisation peut notamment comprendre un LIDAR.

Dans certains modes de réalisation, le module de pilotage comprend un module de suivi configuré pour suivre le patient dans son environnement. Ce module de suivi permet au robot de suivre le patient de manière autonome : le patient possède donc une totale liberté de mouvement au sein de son environnement, le robot étant capable de le suivre dans ses déplacements.

Dans certains modes de réalisation, le module de suivi du patient comprend un module de reconnaissance faciale.

Dans certains modes de réalisation, le système comprend au moins une balise de suivi, configurée pour être portée par le patient et pour être détectée par le module de suivi du robot.

Dans certains modes de réalisation, cette balise de suivi est un motif particulier porté par le patient est reconnu par le module de suivi sur les images enregistrées par la caméra. Une telle balise peut notamment être associée audit au moins un capteur : par exemple, dans le cas d'électrodes EEG, la balise peut être prévue sur le bonnet portant les électrodes.

Dans certains modes de réalisation, cette balise de suivi est une balise émettant un signal à destination du robot. Le module de suivi peut par exemple suivre le signal du boîtier de communication porté le patient, typiquement son signal wifi.

Dans certains modes de réalisation, le module de pilotage est configuré pour recevoir des commandes de pilotage externes. Dans un tel cas, le robot peut être piloté à distance par le serveur distant ou bien en local par une télécommande.

Dans certains modes de réalisation, la caméra est orientable. Ceci permet de suivre le patient sans nécessairement manœuvrer le robot.

Dans certains modes de réalisation, la caméra est située à une hauteur comprise entre 100 et 190 cm, de préférence entre 120 et 180 cm, du sol. Une telle hauteur permet de réduire le risque que la vision de la caméra ne soit obstruée par des meubles par exemple ; elle permet également une bonne vision du patient y compris lorsque ce dernier est allongé sur son lit.

Dans certains modes de réalisation, la caméra est prévue à l'extrémité d'une perche verticale portée par le robot. Ceci est tout particulièrement utile lorsque le châssis du robot possède une hauteur réduite.

Dans certains modes de réalisation, la caméra est prévue sur une tête du robot, la tête du robot étant montée à l'extrémité d'un cou s'étendant verticalement depuis le châssis du robot sur au moins 30 cm, de préférence sur au moins 40 cm. Cette tête peut également porter au moins un écran.

Dans certains modes de réalisation, le système comprend deux caméras distinctes. Une première caméra peut ainsi être dédiée à la synchronisation avec le signal dudit au moins un capteur, par exemple dans le cadre d'un EEG vidéo, tandis que la deuxième caméra peut être dédiée à la surveillance du patient par une équipe médicale et à la communication entre le patient et cette équipe médicale. En particulier, lorsque le robot comprend deux systèmes informatiques distincts, la première caméra peut être connectée au premier système informatique tandis que la deuxième caméra peut être connectée au deuxième système informatique.

Dans certains modes de réalisation, le système comprend au moins un écran, de préférence tactile. Cet écran est utile pour afficher le signal traité par le module de traitement et/ou pour communiquer avec une équipe médicale distante. Il peut également servir à interagir avec le robot.

Dans certains modes de réalisation, le système comprend deux écrans distincts. Un premier écran peut ainsi être dédié à l'affichage du signal traité tandis que le deuxième écran peut être dédié à la communication entre le patient et cette équipe médicale. En particulier, lorsque le robot comprend deux systèmes informatiques distincts, le premier écran peut être connecté au premier système informatique tandis que le deuxième écran peut être connecté au deuxième système informatique.

Dans certains modes de réalisation, le module de traitement est configuré pour synchroniser le signal dudit au moins un capteur avec le signal de ladite au moins une caméra.

Dans certains modes de réalisation, le module de traitement est configuré pour réaliser de manière autonome une analyse au moins partielle du signal dudit au moins un capteur.

Dans certains modes de réalisation, le module de traitement est configuré pour détecter une situation anormale et transmettre une alerte au serveur distant par l'intermédiaire du deuxième module de communication. Une telle alerte est notamment utile pour permettre à une équipe médicale d'observer en direct le comportement du patient durant cette situation anormale, par exemple une crise, et mettre en place une procédure de secours en cas de besoin.

Dans certains modes de réalisation, le deuxième module de communication est capable de se connecter à internet par l'intermédiaire d'une connexion wifi ou de téléphonie mobile, par exemple du type 4G ou5G.

Dans certains modes de réalisation, le deuxième module de communication comprend une mémoire tampon configurée pour conserver les données transmises au serveur distant jusqu'à ce que le serveur distant confirme la bonne réception de ces données. On évite ainsi la perte ou la corruption de données.

Dans certains modes de réalisation, le robot comprend un troisième module de communication, configuré pour communiquer avec une équipe médicale distante. Cette communication peut notamment prendre la forme d'un flux vidéo et/ou audio, unilatéral ou bilatéral. Les deuxième et troisième modules de communication peuvent être communs ou bien distincts. En particulier, lorsque le robot comprend deux systèmes informatiques distincts, le deuxième module de communication peut être connecté au premier système informatique tandis que le troisième module de communication peut être connecté au deuxième système informatique.

Dans certains modes de réalisation, le système comprend au moins une station de recharge, configurée pour recharger une batterie du robot lorsque le robot est connecté à la station de recharge. Ainsi, de préférence, le robot est dépourvu d'un câble d'alimentation, ce qui augmente la liberté de mouvement du robot, et donc du patient. Au moins une station de recharge est prévue dans la chambre du patient, de sorte que le robot puisse continuer la surveillance du patient depuis sa station de recharge, y compris la nuit. De préférence, au moins une deuxième station de recharge est prévue dans une seconde pièce de l'environnement du patient, par exemple un salon, de sorte que le robot puisse être rechargé en journée sans contraindre le patient à rejoindre sa chambre lorsque la batterie du robot est faible.

Dans certains modes de réalisation, le robot comprend un module d'avertissement configuré pour avertir le patient lorsque la batterie du robot est faible et lui demander de se rapprocher d'une station de recharge. De cette manière, le robot peut demander au patient de rejoindre une pièce où le robot pourra être rechargé tout en continuant la surveillance du patient.

Le présent exposé concerne également un procédé de surveillance d'un patient à l'aide d'un système de surveillance selon l'un quelconque des modes de réalisation précédents, comprenant les étapes suivantes :
pose dudit au moins un capteur sur le patient ;
introduction du robot dans l'environnement du patient ; et
analyse des données transmises par le robot au serveur distant.

Comme cela a été expliqué ci-avant au sujet du système de surveillance, un tel procédé de surveillance permet de réaliser une surveillance longue d'un patient de manière beaucoup plus facile, pratique et agile. En particulier, toutes les caractéristiques facultatives présentées ci-dessus au sujet du système de surveillance, ainsi que tous les effets techniques associés, peuvent se transposer directement à ce procédé de surveillance.

Dans certains modes de réalisation, la surveillance a lieu durant plusieurs jours consécutifs, de préférence au moins 3 jours consécutifs, de préférence encore au moins 5 jours consécutifs. De préférence, cette surveillance est réalisée de manière continue, jour et nuit.

Dans certains modes de réalisation, la surveillance a lieu en dehors d'un hôpital ou d'un espace médicalisé.

Dans certains modes de réalisation, la surveillance a lieu au domicile du patient.

Dans certains modes de réalisation, un opérateur rend visite quotidiennement au patient afin de réaliser la maintenance du système. Cette maintenance peut notamment comprendre la vérification et le repositionnement éventuel d'un ou plusieurs capteurs du système ainsi que la vérification et le remplacement éventuel des piles ou des batteries du boîtier de communication. Cette maintenance peut notamment être réalisée dans le cadre de l'hospitalisation à domicile.

Dans certains modes de réalisation, le procédé comprend une étape de visioconférence entre le patient et une équipe médicale par l'intermédiaire du robot. Une telle visioconférence peut être utile pour surveiller le patient en direct, notamment en vue d'observer une crise et/ou mettre en place des secours en cas d'urgence médicale. Un système automatisé de détection peut aussi, le cas échéant envoyer un avertissement ou une alarme sur le téléphone d'un sujet déterminé (parents par exemple).

Dans certains modes de réalisation, au cours de l'étape de visioconférence, l'équipe médicale transmet des exercices au patient et surveille la réalisation des exercices par le patient. De tels exercices peuvent notamment aider l'équipe médicale à établir un diagnostic.

Dans certains modes de réalisation, le procédé comprend une étape d'envoi des secours, déclenchée par l'équipe médicale ou de manière autonome par le robot, lorsque le patient subit une urgence médicale.

Les caractéristiques et avantages précités, ainsi que d'autres, apparaîtront à la lecture de la description détaillée qui suit, d'exemples de réalisation du système de surveillance et du procédé de surveillance proposés. Cette description détaillée fait référence aux dessins annexés.

### Brève description des dessins

Les dessins annexés sont schématiques et visent avant tout à illustrer les principes de l'exposé.

Sur ces dessins, d'une figure à l'autre, des éléments (ou parties d'élément) identiques sont repérés par les mêmes signes de référence. En outre, des éléments (ou parties d'élément) appartenant à des exemples de réalisation différents mais ayant une fonction analogue sont repérés sur les figures par des références numériques incrémentées de 100, 200, etc.
[Fig. 1] La figure 1 est une représentation schématique d'un premier exemple de système de surveillance.
[Fig. 2] La figure 2 est un schéma fonctionnel du premier exemple de système de surveillance.
[Fig. 3] La figure 3 représente un appartement dans lequel est installé le robot du premier exemple de système de surveillance.
[Fig. 4] La figure 4 est un schéma fonctionnel d'un deuxième exemple de système de surveillance.

### Description des modes de réalisation

Afin de rendre plus concret l'exposé, des exemples de systèmes de surveillance et de procédés de surveillance sont décrits en détail ci-après, en référence aux dessins annexés. Il est rappelé que l'invention ne se limite pas à ces exemples.

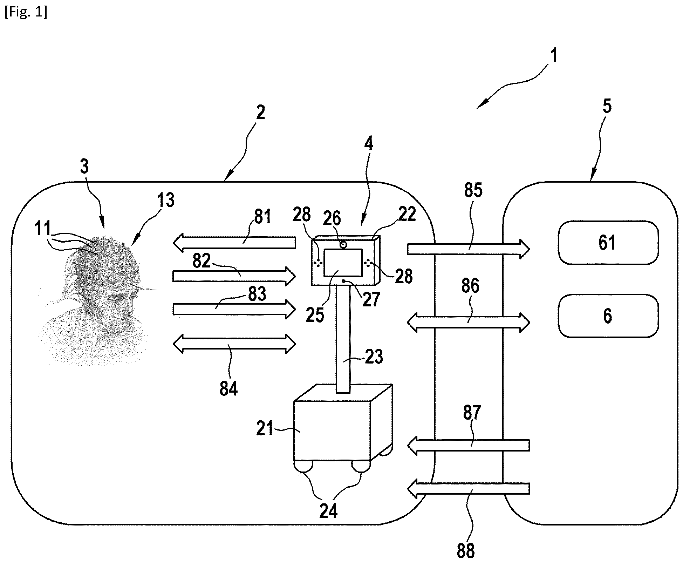

La figure 1 représente, de manière schématique, un premier exemple de système de surveillance 1. Ce système 1 s'articule autour de trois pôles : le patient 3, l'équipe médicale 6 et un robot 4 assurant l'interface entre le patient 3 et l'équipe médicale 6. L'équipe médicale 6 se situe dans un milieu médical, tel qu'un hôpital 5, tandis que le patient 3 et le robot 4 se situent dans un milieu familier du patient 3, typiquement son domicile 2.

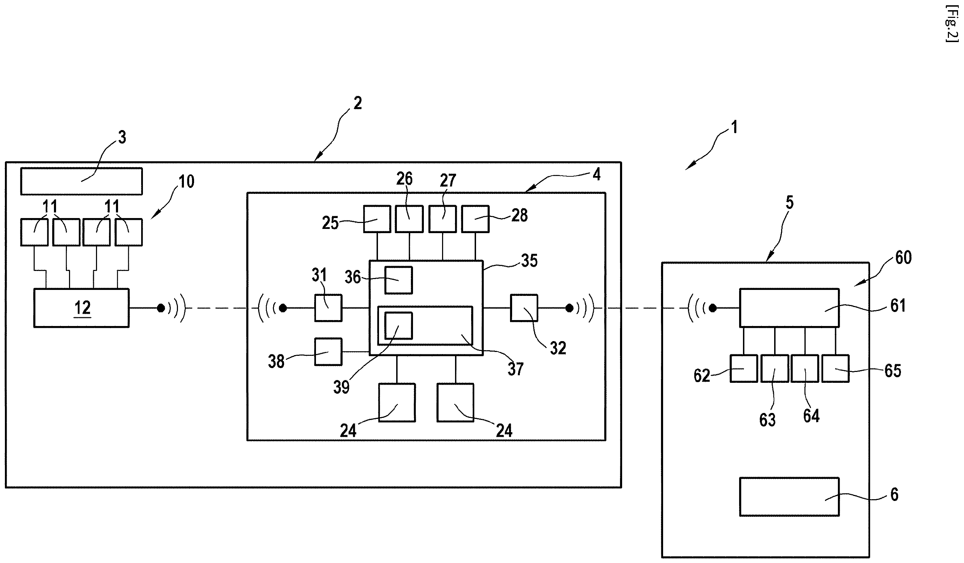

Comme cela est visible sur la figure 2, le système 1 comprend ainsi un premier ensemble 10 destiné à être porté par le patient 3. ce premier ensemble 10 comprend une pluralité de capteurs 11 reliés à un boîtier de communication 12. Dans le présent exemple, les capteurs 11 sont des électrodes d'électroencéphalogramme (EEG), du type cupules, fixées sur le cuir chevelu du patient 3. Les électrodes peuvent aussi être contenues dans un bonnet 13 qui peut porter 21, 27, 32 ou 64 électrodes 11.

Le boîtier de communication 12 comprend une alimentation électrique autonome, typiquement une ou plusieurs piles ou batteries rechargeables. Il est configuré pour recevoir les signaux individuels de chacun des capteurs 11 et de les formater : ce formatage comprend en particulier le regroupement des différents signaux en un fichier unique. Il est également capable d'établir une connexion wifi avec le robot 4 en vue de lui transmettre les signaux ainsi formatés.

Le système 1 comprend ensuite un deuxième ensemble correspondant au robot 4. Le robot 4 comprend un châssis 21 et une tête 22 montée au sommet du châssis 21 par l'intermédiaire d'un cou 23. Le châssis 21 comprend deux roues motrices 24 formant un dispositif de locomotion. La tête 22 est articulée à l'extrémité supérieure du cou 23 autour de deux axes, permettant ainsi à la tête 22 d'être orientée de droite à gauche et de haut en bas. La tête 22 comprend un écran 25, une caméra 26, un microphone 27 et des haut-parleurs 28. L'écran 25 peut éventuellement être tactile. Elle peut également comprendre un dispositif d'éclairage, par exemple du type projecteur. De manière alternative ou en complément, le robot 4 peut comprendre une caméra infra-rouge capable de vision nocturne : il peut s'agir de la caméra 26 ou bien d'une caméra additionnelle.

La hauteur du châssis 21 est de préférence assez faible, de l'ordre de 40 cm, afin de réduire le risque de basculement. Le cou 23 possède pour sa part une longueur de l'ordre de 60 à 120 cm de sorte que la caméra 26 portée par la tête 22 du robot 4 se situe à une hauteur d'environ 120 à 180 cm par rapport au sol.

Le robot 4 comprend en outre un premier module de communication 31, capable de communiquer avec le boîtier de communication 12, de préférence à l'aide d'une connexion wifi, ainsi qu'un deuxième module de communication 32, capable de se connecter à internet, soit par l'intermédiaire d'un réseau de téléphonie mobile, de préférence 4G ou 5G, soit par l'intermédiaire du réseau wifi d'une passerelle domestique (box internet) connectée au réseau internet.

Ces différents organes du robot 4 sont connectés à un système informatique central 35, hébergé de préférence au sein du châssis 21 du robot 4. Le système informatique 35 comprend tous les organes nécessaires à son fonctionnement et, notamment, un processeur, une mémoire vive et au moins un disque de stockage de données.

Le système informatique 35 comprend un module de traitement 36 recevant et traitant les signaux des capteurs 11 transmis par le boîtier de communication 12. En particulier, dans le présent exemple, le module de traitement 36 est un module de traitement d'EEG-vidéo capable de synchroniser les signaux EEG de chaque électrode 11 avec le flux vidéo enregistré par la caméra 26. Il est également capable d'afficher ces signaux EEG sur l'écran 25. Le module de traitement 36 transmet alors le signal ainsi traité au deuxième module de communication 32 afin qu'il puisse être transmis à l'équipe médicale 6 par l'intermédiaire du réseau internet.

Le système informatique 35 comprend également un module de pilotage 37 configuré pour piloter le robot 4 au sein de son environnement, ici le domicile 2 du patient 3. Ce module de pilotage 37 est connecté à au moins un dispositif de spatialisation 38 du robot 4, par exemple un LIDAR, lui permettant de se repérer dans son environnement et, en particulier, d'éviter les obstacles ; ce dispositif de spatialisation 38 permet également d'analyser les mouvements du patient. Il comprend également un module de suivi 39 programmé pour suivre le patient 3 : ce module de suivi 39 peut combiner plusieurs méthodes de suivi, par exemple de la reconnaissance d'image et du suivi de signal, en particulier le signal wifi du boîtier de communication 12. Sur la base des données de spatialisation recueillies par le dispositif de spatialisation 38 et des coordonnées du patient 3 déterminées par le module de suivi 39, le module de pilotage 37 calcule une trajectoire pour suivre le patient 3 et commande les roues 24 en conséquence.

Le système comprend enfin un troisième ensemble 60 situé au sein de l'hôpital 5. Ce troisième ensemble 60 comprend un serveur 61 connecté à internet et donc capable de communiquer avec le robot 4 par l'intermédiaire du réseau internet et du deuxième module de communication 32 du robot 4. L'équipe médicale 6 peut alors consulter le serveur distant 61 afin de récupérer le signal traité par le robot 4 et mener sur ce dernier les analyses utiles en vue notamment d'établir un diagnostic.

Le module de communication 32 transmet également au serveur 61 le flux vidéo enregistré par la caméra 26 du robot 4 ainsi que le flux audio enregistré par le microphone 27 du robot 4 : ces derniers peuvent être diffusés à destination de l'équipe médicale 6 à l'aide d'un écran 62 et d'un haut-parleur 63 connectés au serveur 61. En retour, une caméra 64 et un microphone 65 connectés au serveur 61 permettent d'envoyer un flux vidéo et un flux audio en provenance de l'équipe médicale 6 ; ces derniers peuvent être diffusés à destination du patient 3 à l'aide de l'écran 26 et des haut-parleurs 28 du robot 4.

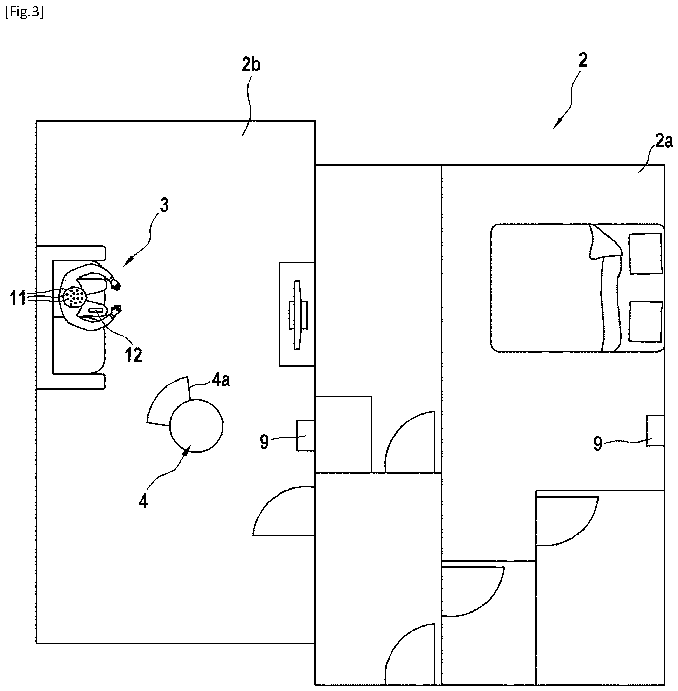

La figure 3 illustre à présent l'intégration du système 1 dans le domicile 2 du patient. Comme cela a été expliqué plus haut, les capteurs 11 sont posés sur le patient 3 et reliés au boîtier de communication 12 qui peut, par exemple, être arrangé dans une sacoche portée en bandoulière. Ces capteurs 11 sont prévus pour être portés en continu, y compris la nuit. Tous les matins, dans le cadre de l'hospitalisation à domicile, un infirmer ou un technicien se rend au domicile 2 du patient 3 et, outre les soins médicaux habituels à pratiquer sur le patient 3, vérifie que les capteurs 11 sont toujours bien positionnés et, le cas échéant, corrige le positionnement des capteurs 11 et réduit les impédances des électrodes par adjonction de pate ou de gel conducteur. En cas de besoin, l'infirmier remplace également les piles du boitier de communication 12.

Par ailleurs, le robot 4 est introduit dans le domicile 2 du patient. Des stations de recharge 9 sont également installées afin de permettre la recharge du robot 4 au cours de sa mission de surveillance qui peut durer plusieurs jours, voire plusieurs semaines. Au moins une station de recharge 9 est installée dans la chambre 2a du patient 3 de sorte que le robot 4 puisse être rechargé durant la nuit tout en continuant de surveiller le patient 3 durant son sommeil. De préférence, une autre station de recharge 9 est également installée dans la pièce à vivre du patient 3, c'est-à-dire la pièce dans laquelle il est supposé passer le plus de temps en journée, typiquement son salon 2b. De cette manière, le patient 3 n'est pas obligé de rejoindre sa chambre 2a en journée si la batterie du robot 4 devient trop faible.

Comme cela a été expliqué plus haut, le robot 4 suit alors le patient 3 pour le garder systématiquement dans son champ de vision 4a, c'est-à-dire le champ de vision de sa caméra 26. De plus, lorsque le robot 4 détermine que sa batterie est faible, il émet un avertissement à destination du patient, par exemple à l'aide de ses haut-parleurs 28, afin d'inviter le patient 3 à rejoindre une pièce dans laquelle se situe une station de recharge 9.

Un procédé de surveillance va maintenant être décrit en référence à la figure 1. Le patient 3 est libre de se déplacer dans son domicile 2 et le robot 4 le suit dans chacun de ses déplacements de sorte que le patient 3 reste dans son champ de vision 4a. Ce suivi est représenté schématiquement à l'aide de la flèche 81.

Le robot 4 filme alors en continu le patient 3, enregistrant le flux vidéo à l'aide de sa caméra 26 et le flux audio à l'aide de son microphone 27. Cet enregistrement vidéo et audio est représenté à l'aide de la flèche 82. De plus, le boîtier de communication 12 transmet en continu au robot 4 les signaux des capteurs 11, ici des électrodes EEG. Cette transmission des signaux EEG est représentée à l'aide de la flèche 83.

Par ailleurs, le patient 3 et le robot 4 peuvent interagir en permanence à l'aide de l'écran 25, de la caméra 26, du microphone 27 et des haut-parleurs 28. En particulier, le patient 3 peut transmettre des ordres au robot 4, soit par commande vocale, soit par commande tactile sur l'écran tactile 25 du robot 4. Cette interaction est représentée à l'aide de la flèche 84. A cet égard, le robot 4 peut se comporter comme un robot compagnon, c'est-à-dire un robot capable d'échanger et/ou de jouer avec le patient 3 : il peut notamment rassurer le patient en se rapprochant de lui et en diffusant des messages d'apaisement en cas de crise. Le robot 4 peut aussi capter des signaux d'urgence (EEG, ECG, Saturation artérielle en Oxygène, Résistances cutanées sympathiques) et générer un signal d'alerte soit aux secours soit sur le téléphone d'un référent (parents par exemple).

Le robot 4 communique par ailleurs en continu avec le serveur distant 61. D'une part, le robot 4 transmet au serveur distant 61 le signal traité, c'est-à-dire dans le présent exemple le signal EEG synchronisé avec le flux vidéo. Cette transmission est représentée à l'aide de la flèche 85. De plus, l'équipe médicale 6 peut à tout moment consulter le flux vidéo et/ou audio enregistré et retransmis par le robot 4. L'équipe médicale 6 peut également initier une communication vidéo et/ou audio avec le patient 3, par exemple sous la forme d'une visioconférence. Une telle communication est représentée à l'aide de la flèche 86.

Par ailleurs, comme cela a été expliqué plus haut, un infirmier ou technicien est envoyé tous les matins au domicile 2 du patient 3 afin de réaliser les soins quotidiens du patient 3 et réaliser la maintenance du système 1. Cette maintenance est représentée à l'aide de la flèche 87. De plus, en cas d'urgence, l'équipe médicale 6 peut envoyer des secours au domicile 2 du patient 3 : ceci est représenté à l'aide de la flèche 88.

La surveillance du patient 3 dure ainsi plusieurs jours, voire plusieurs semaines, jusqu'à ce que le patient ait fait un nombre suffisant de crises. A cet égard, le traitement médicamenteux du patient peut être modifié ou suspendu de manière à favoriser l'apparition de crises.

Au cours de chaque crise, les signaux EEG et le flux vidéo sont enregistrés de manière synchrone et transmis au serveur distant 61. L'équipe médical 6 peut alors analyser les signaux EEG correspondants à la crise ainsi que la séquence vidéo associée en vue notamment d'établie le diagnostic du patient 3. En cas de crise particulièrement grave, l'équipe médicale 6 peut communiquer avec le patient 3 et, éventuellement envoyer des secours au domicile 2 du patient 3.

L'équipe médicale 6 peut également transmettre des exercices au patient 2 par l'intermédiaire du robot 4 et surveiller leur réalisation. Ceci peut également aider à établir le diagnostic du patient.

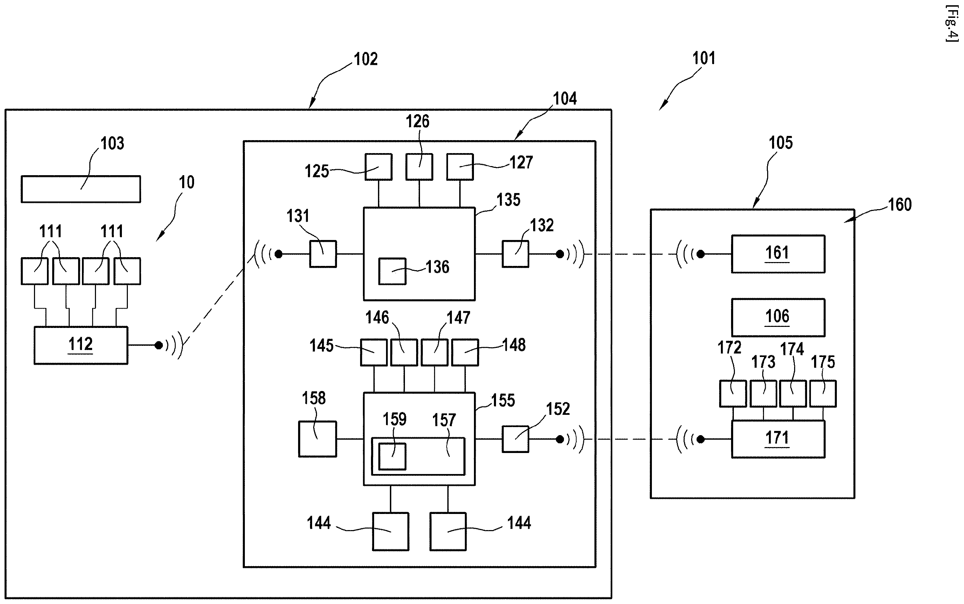

Un deuxième exemple de système de surveillance 101 est représenté sur la figure 4. Seules les différences avec le premier exemple seront décrites. La principale différence de ce deuxième exemple réside dans le fait que le robot 104 comprend deux systèmes informatiques 135, 155 distincts au lieu d'un seul.

Le premier système informatique 135, pouvant par exemple fonctionner sous Windows, est responsable du traitement du signal EEG-vidéo.

Le premier système informatique comprend donc un module de traitement 136 analogue à celui du premier exemple ; il connecté à un premier module de communication 131, analogue à celui du premier exemple, pour réceptionner le signal EEG et à un deuxième module de communication 132, analogue à celui du premier exemple, pour retransmettre le signal traité au serveur distant 161.

Dans cet exemple, le robot 104 comprend un premier écran 125, une première caméra 126 et d'un premier microphone 127, tous connectés au premier système informatique 135 : le premier écran 125 est prévu pour afficher le signal EEG tandis que la première caméra 126 et le premier microphone 127 sont dédiés à l'enregistrement de la vidéo qui sera synchronisée avec le signal EEG.

Le deuxième système informatique 155, pouvant par exemple fonctionner sous Android, est responsable du fonctionnement de toutes les autres fonctions du robot 104, en particulier sa locomotion, son interaction avec le patient 103 et la communication avec l'équipe médicale 106.

Le deuxième système informatique 155 comprend donc un module de pilotage 157 et un module de suivi 159, analogues à ceux du premier exemple, connectés à un dispositif de spatialisation 158, analogue à celui du premier exemple, et configuré pour commander les roues 144 du robot 104.

Dans cet exemple, le robot 104 comprend également un deuxième écran 145, une deuxième caméra 146, un deuxième microphone 147 et des haut-parleurs 148, tous connectés au deuxième système informatique 155. Ces dispositifs sont dédiés à l'interaction avec le patient 103 et à la communication avec l'équipe médicale 106. Pour cela, le robot 104 comprend dans cet exemple un troisième module de communication 152, connecté au deuxième système informatique 155, capable de se connecter, par l'intermédiaire du réseau internet, à un dispositif de visioconférence 171 de l'hôpital 105.

La configuration de ce deuxième exemple est particulièrement adaptée dans le cas d'une mise à niveau d'un robot préexistant. Dans un tel cas, le deuxième système informatique 155 peut être le système informatique natif du robot 104 et l'ensemble des périphériques qui y sont connectés peuvent être les périphériques natifs du robot 104. Dans un tel cas, le premier système informatique 135 et l'ensemble des périphériques qui y sont connectés peuvent être rapportés sur le robot préexistant. Par exemple, le premier système informatique peut être intégré dans un boîtier rapporté sous ou sur un côté du châssis du robot 104.

Naturellement, d'autres configurations, éventuellement à mi-chemin entre le premier et le deuxième exemple, peuvent être imaginées. Par exemple, le robot peut comprendre deux systèmes informatiques distincts mais partageant certains périphériques : en particulier, une caméra unique peut être connectée aux deux systèmes et servir à la fois à la synchronisation du signal EEG et à la communication avec l'équipe médicale ; un écran unique peut également être prévu, avec éventuellement deux entrées distinctes. Dans un autre exemple, l'un des deux systèmes informatiques peut encapsuler l'autre système informatique.

De même, au lieu de prévoir au sein de l'hôpital un serveur 161 dédié à la récupération du signal EEG vidéo et un dispositif de visio-conférence 171 distinct, il est possible d'imaginer que toutes ces fonctions sont intégrées dans un serveur unique, à la manière du premier exemple, connecté à la fois avec le deuxième et le troisième module de communication 132, 152 du robot 104.

En tout état de cause, quelle que soit la configuration du robot, son fonctionnement reste analogue, de telle sorte que le procédé de surveillance décrit plus haut reste inchangé.

Bien que la présente invention ait été décrite en se référant à des exemples de réalisation spécifiques, il est évident que des modifications et des changements peuvent être effectués sur ces exemples sans sortir de la portée générale de l'invention telle que définie par les revendications. En particulier, des caractéristiques individuelles des différents modes de réalisation illustrés/mentionnés peuvent être combinées dans des modes de réalisation additionnels. Par conséquent, la description et les dessins doivent être considérés dans un sens illustratif plutôt que restrictif.

Il est également évident que toutes les caractéristiques décrites en référence à un procédé sont transposables, seules ou en combinaison, à un dispositif, et inversement, toutes les caractéristiques décrites en référence à un dispositif sont transposables, seules ou en combinaison, à un procédé.

## Revendications

1. Système de surveillance d'un patient, comprenant au moins un capteur (11), configuré pour être posé sur le patient (3) et pour enregistrer au moins un signal relatif au patient (3), au moins un capteur étant une électrode d'électroencéphalogramme, et
un robot (4), comportant
- un dispositif de locomotion (24), commandé par un module de pilotage (37),
- au moins une caméra (26),
- un premier module de communication (31), configuré pour recevoir, de manière sans fil, le signal dudit au moins un capteur (11),
- un deuxième module de communication (32), configuré pour communiquer avec un serveur distant (61), et
- un module de traitement (36), configuré pour traiter le signal dudit au moins un capteur (11) et fournir un signal traité au deuxième module de communication (32).

2. Système selon la revendication 1, dans lequel ledit au moins un capteur comprend au moins un jeu d'électrodes d'électroencéphalogramme (11) comportant au moins 8 électrodes (11) et de préférence au moins 32 électrodes (11).

3. Système selon la revendication 1 ou 2, comprenant un boîtier de communication (12) connecté audit au moins un capteur (11) et configuré pour communiquer avec le premier module de communication (31) du robot (4).

4. Système selon l'une quelconque des revendications 1 à 3, dans lequel le robot (104) comprend un premier système informatique (135), incluant le premier module de communication (131) et le module de traitement (136), et un deuxième système informatique (155), distinct du premier système informatique (135), incluant le module de pilotage (157).

5. Système selon la revendication 4, dans lequel le robot (104) comprend un châssis (21), le premier système informatique (135) étant intégré à l'intérieur du châssis (21) tandis que le deuxième système informatique (155) est rapporté sous le châssis (21) ou sur un côté du châssis (21).

6. Système selon l'une quelconque des revendications 1 à 5, dans lequel le module de pilotage (157) comprend un module de suivi (159) configuré pour suivre le patient (3) dans son environnement (2).

7. Système selon l'une quelconque des revendications 1 à 6, dans lequel la caméra (26) est située à une hauteur comprise entre 100 et 190 cm, de préférence entre 120 et 180 cm, du sol.

8. Système selon l'une quelconque des revendications 1 à 7, dans lequel le module de traitement (36) est configuré pour synchroniser le signal dudit au moins un capteur (11) avec le signal de ladite au moins une caméra (26), le robot (4) étant configuré pour filmer en continu le patient (3).

9. Système selon l'une quelconque des revendications 1 à 8, dans lequel le robot (4) comprend un troisième module de communication (32), configuré pour communiquer avec une équipe médicale distante (6).

10. Système selon l'une quelconque des revendications 1 à 9, comprenant au moins une station de recharge (9), configurée pour recharger une batterie du robot (4) lorsque le robot (4) est connecté à la station de recharge (9), et dans lequel le robot (4) comprend un module d'avertissement configuré pour avertir le patient (3) lorsque la batterie du robot (4) est faible et lui demander de se rapprocher d'une station de recharge (9).

11. Procédé de surveillance d'un patient à l'aide d'un système de surveillance selon l'une quelconque des revendications 1 à 10, comprenant les étapes suivantes :
pose dudit au moins un capteur (11) sur le patient (3) ;
introduction du robot (4) dans l'environnement (2) du patient (3) ; et
analyse des données transmises par le robot (4) au serveur distant (61).

12. Procédé selon la revendication 11, dans lequel la surveillance a lieu durant plusieurs jours consécutifs, de préférence au moins 3 jours consécutifs, de préférence encore au moins 5 jours consécutifs.

13. Procédé selon la revendication 11 ou 12, dans lequel la surveillance a lieu en dehors d'un hôpital ou d'un espace médicalisé, de préférence au domicile (2) du patient (3).

14. Procédé selon l'une quelconque des revendications 11 à 13, comprenant une étape de visioconférence entre le patient (3) et une équipe médicale (6) par l'intermédiaire du robot (4).
